(19) Europäisches Patentamt / European Patent Office / Office européen des brevets

(11) **EP 3 261 056 A1**

(12) **EUROPÄISCHE PATENTANMELDUNG**

(43) Veröffentlichungstag:
**27.12.2017 Patentblatt 2017/52**

(21) Anmeldenummer: **16176087.1**

(22) Anmeldetag: **24.06.2016**

(51) Int Cl.:
*G06T 7/00* (2017.01)          *A61B 5/107* (2006.01)
*A61B 5/00* (2006.01)

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**
Benannte Validierungsstaaten:
**MA MD**

(71) Anmelder: **Justus-Liebig-Universität Gießen
35390 Gießen (DE)**

(72) Erfinder: **Wilbrand, Jan-Falco
35392 Gießen (DE)**

(74) Vertreter: **Stumpf, Peter
c/o TransMIT GmbH
Kerkrader Strasse 3
35394 Gießen (DE)**

(54) **VERFAHREN ZUR MESSUNG UND EINSTUFUNG DER SCHÄDELFORM**

(57) Gegenstand der Erfindung ist ein Verfahren zur Messung und Einstufung der Schädelform, wobei der zu untersuchende Kopf 1 zunächst optisch erfasst wird. Anschließend werden Schädellänge SL und Schädelbreite SB gemessen und daraus der Cranial Index CI bestimmt. Anschließend wird mindestens ein Paar Schädeldiagonalen festgelegt und deren Länge bestimmt. Aus der Länge der Schädeldiagonalen wird dann der Cranial Vault Asymmetry Index bestimmt und aus dem Vergleich der Maximalwerte von Cranial Index CI und Cranial Vault Asymmetry Index eine Einstufung der Schädelform vorgenommen.

EP 3 261 056 A1

## Beschreibung

Die vorliegende Erfindung betrifft ein Verfahren Messung und Einstufung der Schädelform hinsichtlich dem Vorliegen einer Schädeldeformität insbesondere bei Säuglingen und Klein(st)kindern von unter 36 Monaten.

## Beschreibung und Einleitung des allgemeinen Gebietes der Erfindung

Seit der Empfehlung der kinderärztlichen Gesellschaften zur ausschließlichen Rückenlagerung von Säuglingen innerhalb des ersten Lebensjahres nehmen kindliche Schädeldeformitäten ("Liegeschädeldeformitäten") massiv an Zahl zu. Aktuell gehen Fachleute von einer Häufigkeit von 1:60 aus. Dies bedeutet, dass eine erste Erfassung der kindlichen Kopfform aktuell sehr häufig notwendig wird, um behandlungsbedürftige Schädeldeformitäten zu erkennen und einer Therapie zuzuführen. Ca. 30% aller Säuglinge zeigen in den ersten 2 Monaten eine passagere Rumpfasymmetrie, die sich auch auf die Schädelform auswirken kann Die Umsetzung der Empfehlung, Säuglinge auf dem Rücken zu lagern, führte zu einem deutlichen Rückgang der plötzlichen Todesfälle (SIDS). Ob diese "back to sleep" Kampagne zu einem höheren Prozentsatz an Lagerungsasymmetrien geführt hat, wird kontrovers diskutiert. Die Berichte einer Zunahme der Lagerungsplagiozephali und insbesondere Brachyzephali überwiegen allerdings deutlich, wobei kontrollierte Daten hierzu fehlen. Studien aus Indien, Korea und den Philippinen haben ebenfalls gezeigt, dass in Kulturen, in denen schon seit längerem eine strikte Rückenlagerung von Säuglingen während des Schlafes empfohlen wird, ein höherer Anteil von occipital abgeflachten Schädeln im Säuglingsalter anzutreffen ist. Allerdings herrscht in diesen Kulturen in Bezug auf die Definition dessen, was als brachyzephal und was als noch normal anzusehen ist, eine andere Sichtweise.

## Stand der Technik

Daten zur anthropometrischen Schädelvermessung werden an spezialisierten Zentren erhoben. Dabei unterscheiden sich die Messmethoden der einzelnen Zentren deutlich voneinander. Es kommen manuelle anthropometrische Messungen der Schädellänge, -breite und der Diagonalen mit Maßband und Messzirkel zur Anwendung. Dies erfordert in diesen Messungen geschultes Personal. Alternativ sind Schädelmessungen mit 3D-Photogrammetrie möglich, was einen sehr hohen apparativen Aufwand erfordert. Ebenfalls sind Schädelmessungen mit Laser, mittels thermoplastischen oder silikonbasierten Kurvenlinealen möglich.

Es erfolgt aktuell die Vorstellung von Kindern, bei denen eine behandlungsbedürftige Schädeldeformität vermutet wird, zunächst bei diversen medizinischen und paramedizinischen Fachleuten, bis schließlich die Überweisung an ein spezialisiertes Zentrum erfolgt. Hier wird die Schädelform i.d.R. mit einer der o.g. Schädelmessmethoden erfasst und ggf. therapeutische Maßnahmen eingeleitet. Für Betroffene sind damit bisher ein langer Anfahrtsweg und z.T. lange Wartezeiten auf Termine verbunden. Weiterhin kann die Entwicklung / der klinische Verlauf einer Schädeldeformität während einer Therapie nur bei neuerlichen Vorstellungsterminen im spezialisierten Zentrum erneut erfasst werden.

All den bislang verwendeten Messmethoden ist gemeinsam, dass eine universelle und ortsunabhängige Erfassung der Schädelform nicht möglich ist. Die wissenschaftliche Auswertung der Daten aus unterschiedlichen Behandlungszentren folgt uneinheitlichen Messprotokollen und lässt so nur eine begrenzte Vergleichbarkeit zu. Dies bedingt Mängel in der Studienlage zum Thema. Eine erste Einschätzung der Schädelform, aber auch die Kontrolle des klinischen Verlaufs bleibt Standort- oder Fachpersonalgebunden.

## Aufgabe

Aufgabe der vorliegenden Erfindung ist es ein einfaches und universell einsetzbares Verfahren zur Messung und Einstufung der Schädelform bereitzustellen.

## Lösung der Aufgabe

Gelöst wird diese Aufgabe durch ein Verfahren mit den Merkmalen des Anspruchs 1. Vorteilhafte Ausführungsformen und Weiterbildungen der vorliegenden Erfindung ergeben sich aus den Unteransprüchen.

Im Folgenden ist mit "vorne" die Kopfseite gemeint, an der sich die Nase befindet. Die davon abgewandte Seite des Kopfes wird im Folgenden als "hinten" bezeichnet.

Eine wichtige Messgröße ist dabei, der kraniale Index oder cranial Index. Dieser ist definiert als Verhältnis der maximalen Breite des Kopfes eines Organismus (Mensch oder Tier) und seiner maximalen Länge multipliziert mit 100.

Ein Messwert für die Einstufung der Schädelform hinsichtlich dem Vorliegen einer Schädeldeformität ist der sogenannte Cranial Vault Asymmetry Index (CVAI). Zur Berechnung dieses Wertes werden zwei Diagonalen auf den knöchernen Schädel projiziert, die durch den Kreuzungspunkt von Längs- und Querdurchmesser des Schädels gehen und jeweils üblicherweise um 30 Grad vom Längsdurchmesser des Schädels abweichen. Die Längendifferenz dieser beiden Diagonalen wird durch die größere Diagonalenlänge dividiert, daraus ergibt sich dann der sogenannte CVAI.

Das erfindungsgemäße Verfahren umfasst wenigstens die folgenden Schritte:

a) optischen Erfassung des zu untersuchenden Kopfes 1, wobei die Erfassung von oben erfolgt und die Nase und beide Ohren auf dem resultierenden Bild erkennbar sind

Die optische Erfassung erfolgt dabei beispielsweise mit einer Digitalkamera. Dadurch liegen die Bildinformationen digital vor, sodass die weiteren verfahrensschritte mit einem Computerprogramm durchgeführt werden können. Bevorzugt ist hier eine 2-dimensonale optische Erfassung. Eine 3 dimensionale Erfassung ist möglich, aber nicht notwendig.

b) Markierung der Positionen 10,20, an denen je ein Ohr am Kopf 1 ansetzt, wobei der Abstand zwischen diesen Positionen die Schädelbreite SB darstellt

c) Markierung der Position 30 an der die Nase am Kopf 1 ansetzt

d) Festlegung der Strecke NH (Nase-Hinterkopf) senkrecht zur Strecke von Position 10 zu Position 30 von Position 20 aus

e) Bestimmung einer Position 40, wo die Strecke NH die Begrenzung des Hinterkopfs schneidet

f) Messung des Abstandes der Positionen 20 und 40, wobei der Abstand zwischen diesen Positionen die Schädellänge SL darstellt

g) Berechnung des Cranial Index (CI), wobei gilt:

$$CI = \frac{SB}{SL} * 100$$

h) Festlegung eines ersten Paares Schädeldiagonalen SD$\alpha$1 und SD$\alpha$2, wobei der Winkel $\alpha$ zwischen NH und SD$\alpha$1 bzw. NH und SD$\alpha$2 zwischen 10° und 60° beträgt

i) Bestimmung der Positionen 50 und 60 als Schnittpunkte der Schädeldiagonalen SD$\alpha$1 und SD$\alpha$2 mit der vorderen Kopfhälfte und Bestimmung der Positionen 70 und 80 als Schnittpunkte der Schädeldiagonalen SD$\alpha$1 und SD$\alpha$2 mit der hinteren Kopfhälfte

j) Messung des Abstandes der Positionen 50 und 70 als Schädeldiagonale S$\alpha$_Diag A und Messung des Abstandes der Positionen 60 und 80 als Schädeldiagonale S$\alpha$_Diag B

k) Berechnung des Cranial Vault Asymmetry Index (CVAI$\alpha$), wobei

$$CVAI\alpha = \frac{|S\alpha\_Diag\_A - S\alpha\_Diag\_B|}{S\alpha\_Diag\_A} x100$$

l) Berechnung des Diagonalenverhältnisses Diag_Ratio_$\alpha$ als Quotienten (S$\alpha$_Diag A / S$\alpha$_Diag B)

m) Vergleich der erhobenen Messwerte für den Cranial Index, sowie jeweils den höchsten Wert des Cranial Vault Asymmetry Index und des Diagonalen Verhältnisses für die verschiedenen Paare von Diagonalen mit Normwerten

n) Einstufung der Schädelform je nach Abweichung von den Normwerten für den Cranial Index, den Cranial Vault Asymmetry Index und das Diagonalenverhältnis Diag_Ratio

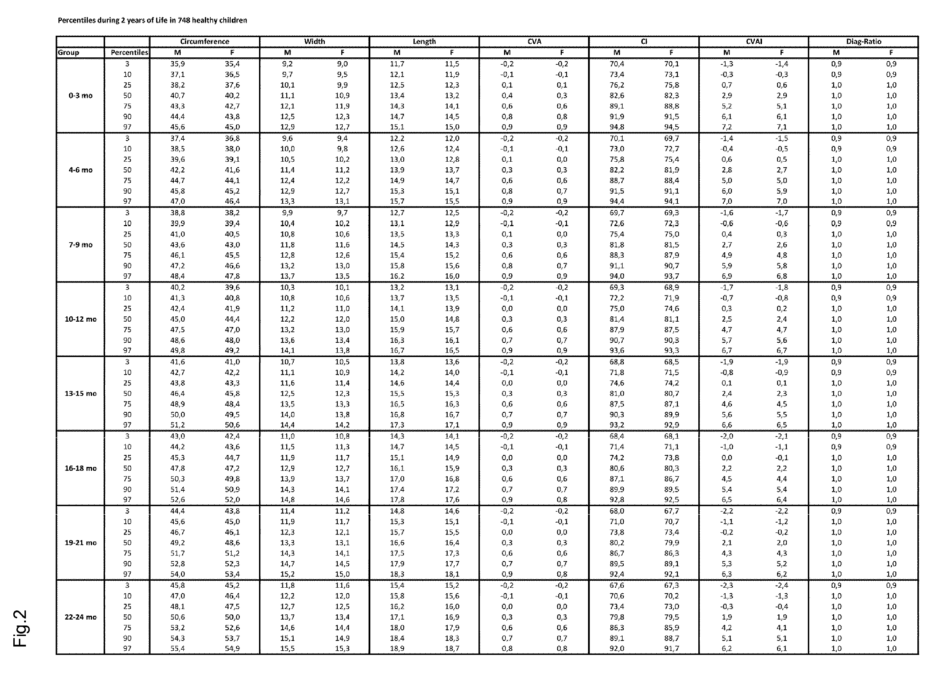

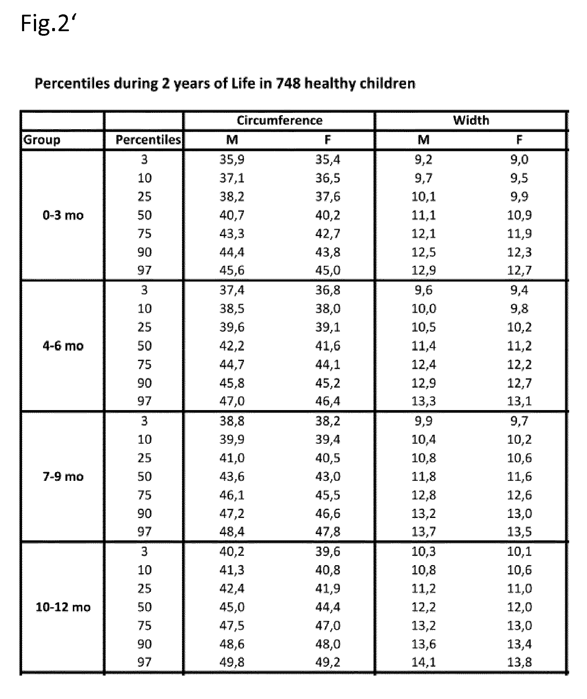

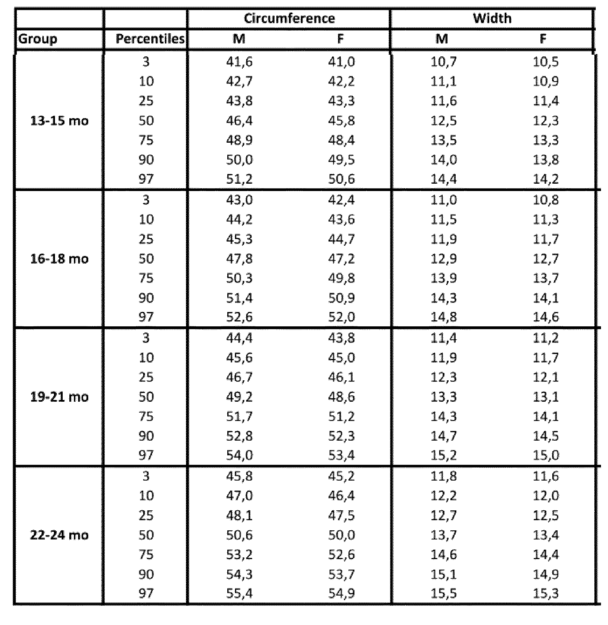

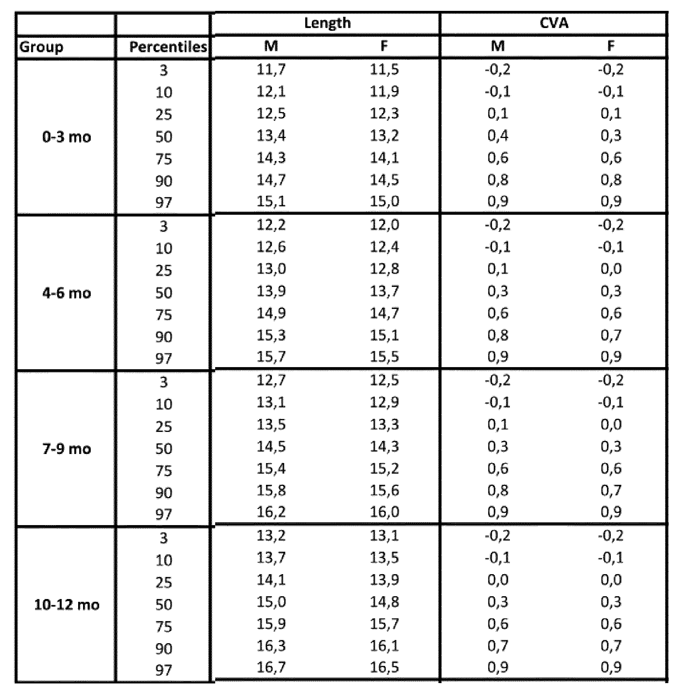

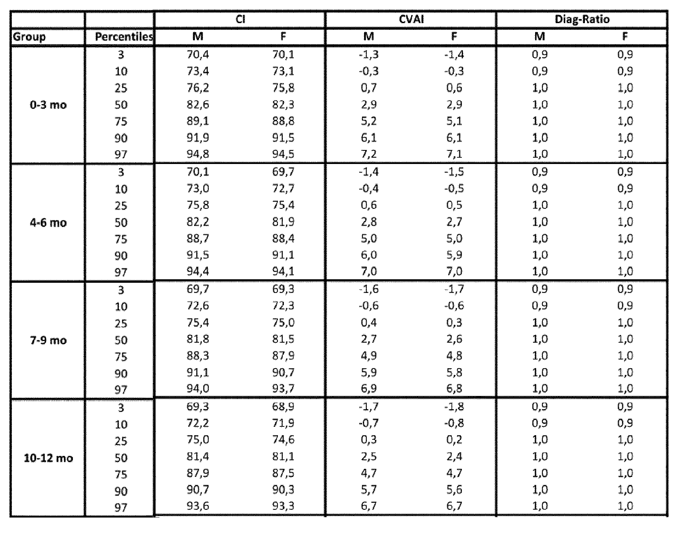

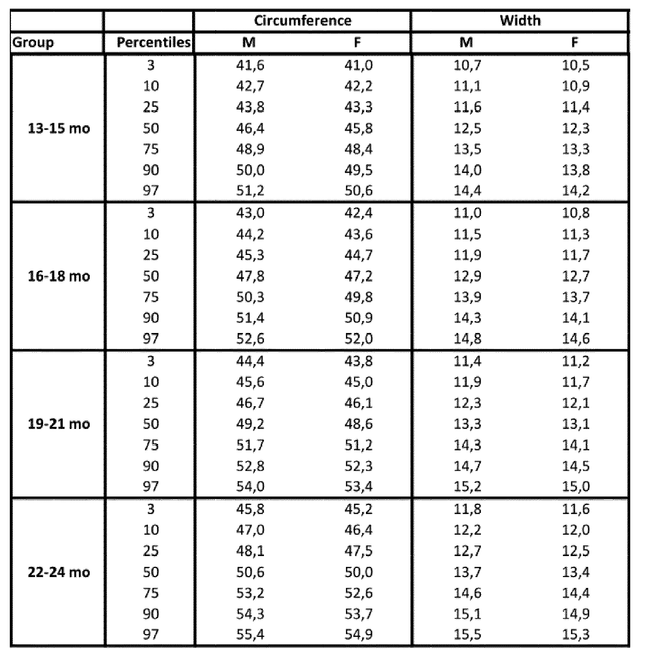

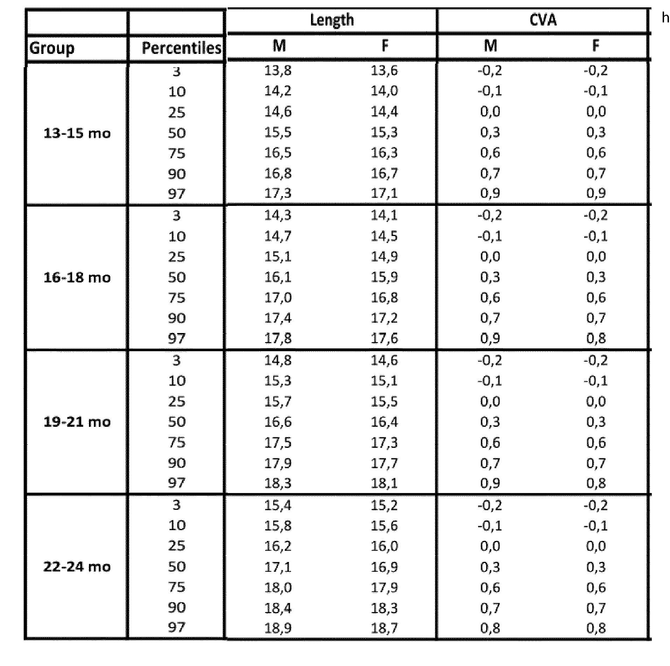

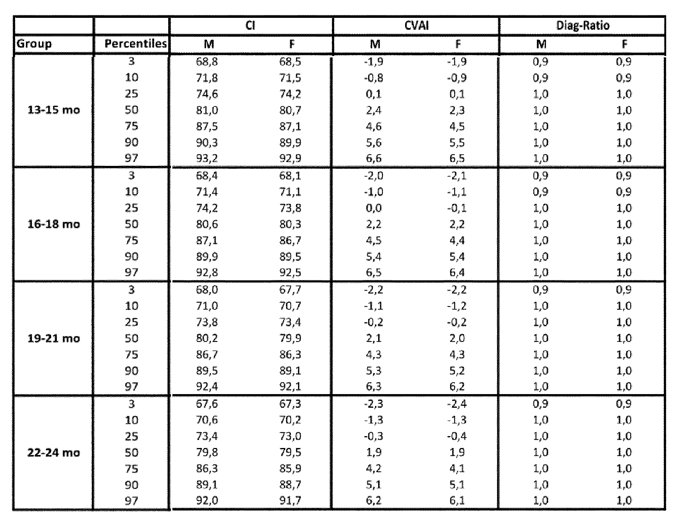

Die hierdurch automatisiert erhobenen Messwerte werden dann mit alters- und geschlechtsgebundenen Normwerten für die entsprechenden Parameter verglichen, um eine Einschätzung der Schädelform bezogen auf eine mögliche Schädeldeformität insbesondere von deren Schwere zu erhalten. Beispielhafte Normwerte sind in der Tabelle aus Fig. 2 zu entnehmen. Dieser Wert kann sich natürlich durch neuere medizinische Erkennnisse auch ändern. Dies hat aber aber keinen Einfluss auf das Verfahren an sich.

In einer zweiten Ausführungsform wird nach Durchführung des Schrittes I), ein zweites Paares Schädeldiagonalen SD$\beta$1 und SD$\beta$2 festgelegt, wobei der Winkel $\beta$ zwischen NH und SD$\beta$1 und SD$\beta$2 zwischen 10° und 60° beträgt und ungleich $\alpha$ ist. Anschrießend werden die Schritte i) bis I) für SD$\beta$1 und SD$\beta$2 durchgeführt und so S$\beta$_Diag A, S$\beta$_Diag B, CVAI$\beta$, Diag_Ratio_$\beta$ bestimmt.

In einer dritten Ausführungsform wird nach Durchführung des Schrittes I), ein drittes Paares Schädeldiagonalen SD$\gamma$1 und SD$\gamma$2 festgelegt, wobei der Winkel $\gamma$ zwischen NH und SD$\gamma$1 und SD$\gamma$2 zwischen 10° und 60° beträgt und ungleich $\alpha$ und $\beta$ ist. Anschließend werden die Schritte i) bis I) für SD$\gamma$1 und SD$\gamma$2 durchgeführt und so S$\gamma$_Diag A, S$\gamma$_Diag B, CVAI$\gamma$, Diag_Ratio_$\beta$ bestimmt.

Es ist möglich ein oder mehrere zusätzliche Paare von Schädeldiagonalen festzulegen und die Längen der Diagonalen, den Cranial Vault Asymmetry Index (CVAI) und das Diagonalenverhältnis Diag_Ratio für weitere Winkel zu bestimmen.

Die Vermessung weiterer Diagonalen hat den Vorteil, dass so auch Deformitäten, welche kleinere Bereiche des Kopfes umfassen besser erfasst werden können. Geeignete Werte für $\alpha$, $\beta$ und $\gamma$ sind 30°, 40° und 50°. Der Wert von $\alpha$ ergibt sich aus gängigen Definition des Cranial Vault Asymmetry Index (CVAI), andere Werte sind möglich.

Die Schädelform wird somit bevorzugt als alters- und geschlechtsentsprechend in wenigstens eine von 3 Kategorien "normal", "moderat deformiert" oder "schwer deformiert" eingestuft. Eine feinere Unterteilung ist möglich.

Eine mögliche Einstufung ist es die Schädelform erfolgt aus dem Vergleich wenigstens eines Kopfparameter CI, CVAI und/oder Diag_Ratio mit Vergleichswerten wie sie beispielhaft in der Tabelle in Fig.2 gezeigt werden.

So ist es möglich eine Schädelform als normal zu einzustufen wenn die Werte der Kopfparameter zwischen der 10% und der 90% Percentile liegen, d.h. wenn die Kopfparameter im Bereich der mittleren 80% liegen. Liegt wenigstens ein Wert außerhalb der der mittleren 80% aber zwischen der 3% und der 97% Percentile wird die Schädelform "moderat deformiert" eingestuft. Wenn ein Kopfparameter außerhalb dieses Bereiches liegt erfolgt eine Einstufung der Schädelform als "schwer deformiert". Diese Einstufung ersetzt keine ärztliche Diagnose, sondern gibt dem Benutzer nur einen Hinweis darauf, dass es sinnvoll sein kann eine solche einzuholen. Des Weiteren kann das Verfahren ergänzend zur Überwachung einer Therapie eingesetzt werden.

Nach einem weiteren Aspekt betrifft die Erfindung ferner ein Computerprogramm zur Verfahren zur Messung und Einstufung von Schädeldeformitäten unter Verwendung eines zuvor beschriebenen Verfahrens. Das Computerprogramm ist insbesondere in einer Auswerteeinheit implementierbar und dient der rechnerischen bzw. computergestützten Analyse der mittels der optischen Erfassung aufgenommenen Bilder. Das Computerprogramm weist insoweit Programmmittel zum eindeutigen Zuordnen der Positionen 10,20,30,40,50,60,70,80 im aufgenommenen Bild auf. Des Weiteren ist das Computerprogramm mit Programmmitteln zum Berechnen der Position und der Lage des Objekts im Raum versehen, welche Programmmittel dazu ausgestaltet sind, die Lage und die Abstände der Positionen 10,20,30,40,50,60,70,80 zu berechnen. Das Computerprogramm stellt insbesondere eine rechnergestützte Umsetzung des Verfahrens zur Messung und Einstufung der Schädelform dar. Insoweit gelten sämtliche zum vorbeschriebenen Verfahren genannten Merkmale, Vorteile und Verfahrensschritte auch in gleicher Art und Weise für das Computerprogramm und umgekehrt.

**Abbildungslegende**

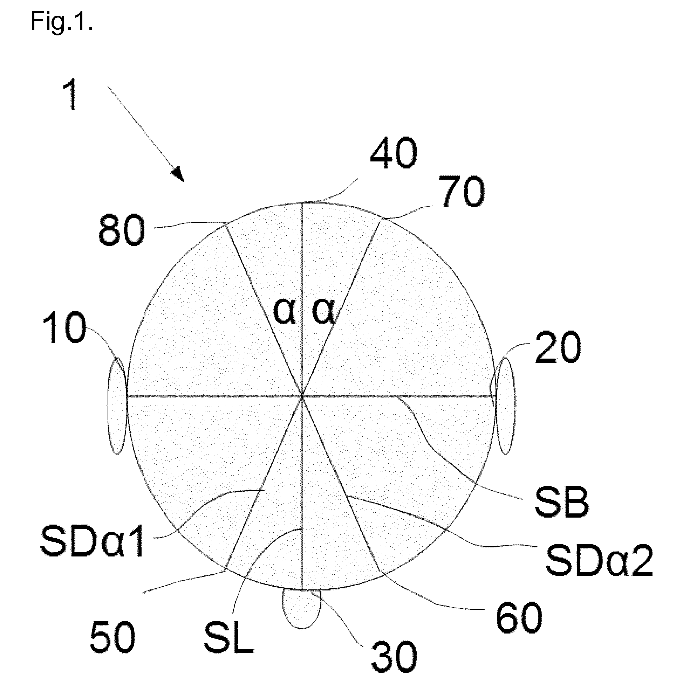

Fig. 1 Schematische Darstellung eines zu untersuchenden Kopfes
Fig.2 Normwerte für die Kopfgrößenparameter bei Klein(st)kindern Die Längenangaben sind hier in cm angegeben.

**Patentansprüche**

1. Verfahren zur Messung und Einstufung der Schädelform, umfassend wenigstens die folgenden Schritte:

   a) optischen Erfassung des zu untersuchenden Kopfes (1), wobei die Erfassung von oben erfolgt und die Nase und beide Ohren auf dem resultierenden Bild erkennbar sind
   b) Markierung der Positionen (10,20), an denen je ein Ohr am Kopf (1) ansetzt, wobei der Abstand zwischen diesen Positionen die Schädelbreite SB darstellt
   c) Markierung der Position (30) an der die Nase am Kopf (1) ansetzt
   d) Festlegung der Strecke NH senkrecht zur Strecke von Position (10) zu Position (30) von Position (20) aus
   e) Bestimmung einer Position (40), wo der Strecke NH die Begrenzung des Hinterkopfs schneidet
   f) Messung des Abstandes der Positionen 20 und 40, wobei der Abstand zwischen diesen Positionen die Schädellänge SL darstellt
   g) Berechnung des Cranial Index CI, wobei CI = SB/SL *100
   h) Festlegung wenigstens eines ersten Paares Schädeldiagonalen SD$\alpha$1 und SD$\alpha$2, wobei der Winkel $\alpha$ zwischen NH und SD$\alpha$1 bzw. NH und SD$\alpha$2 zwischen 10° und 50° beträgt
   i) Bestimmung der Positionen (50) und (60) als Schnittpunkte der Schädeldiagonalen SD$\alpha$1 und SD$\alpha$2 mit der vorderen Kopfhälfte und Bestimmung der Positionen (70) und (80) als Schnittpunkte der Schädeldiagonalen SD$\alpha$1 und SD$\alpha$2 mit der hinteren Kopfhälfte
   j) Messung des Abstandes der Positionen (50) und (70) als Schädeldiagonale S$\alpha$_Diag A und Messung des Abstandes der Positionen 60 und 80 als Schädeldiagonale S$\alpha$_Diag B
   k) Berechnung des Cranial Vault Asymmetry Index (CVAI$\alpha$), wobei gilt:

$$CVAI\alpha = \frac{|S\alpha\_Diag\_A - S\alpha\_Diag\_B|}{S\alpha\_Diag\_A}x100$$

l) Berechnung des Diagonalenverhältnisses Diag_Ratio_$\alpha$ als Quotienten (S$\alpha$_Diag A / S$\alpha$_Diag B)

m) Vergleich der erhobenen Messwerte für den Cranial Index CI, sowie jeweils den höchsten Wert des Cranial Vault Asymmetry Index CVAI und des Diagonalen Verhältnisses für das Diagonalenpaar/die Diagonalenpaare

n) Einstufung der Schädelform je nach Abweichung von den Normwerten für den Cranial Index, den Cranial Vault Asymmetry Index und das Diagonalenverhältniss Diag_Ratio

2. Verfahren, gemäß Anspruch 1, **dadurch gekennzeichnet dass** nach Durchführung des Schrittes l), ein zweites Paares Schädeldiagonalen SD$\beta$1 und SD$\beta$2 festgelegt wird , wobei der Winkel $\beta$ zwischen NH und SD$\beta$1 und SD$\beta$2 zwischen 10° und 60° beträgt und ungleich $\alpha$ ist und anschließend die Schritte i) bis l) für SD$\beta$1 und SD$\beta$2 durchgeführt werden.

3. Verfahren, gemäß Anspruch 2, **dadurch gekennzeichnet dass** nach Durchführung des Schrittes l), ein drittes Paares Schädeldiagonalen SD$\gamma$1 und SD$\gamma$2 festgelegt wird , wobei der Winkel $\gamma$ zwischen NH und SD$\gamma$1 und SD$\gamma$2 zwischen 10° und 60° beträgt und ungleich $\gamma$ und $\beta$ ist und anschließend werden die Schritte i) bis l) für SD$\gamma$1 und SD$\gamma$2 durchgeführt.

4. Computerprogramm zur Messung und Einstufung der Schädelform, zur Durchführung eines Verfahrens gemäß einem der vorhergehenden Ansprüche.

Fig.1.

## Fig.2

Percentiles during 2 years of Life in 748 healthy children

| Group | Percentiles | Circumference | | Width | | Length | | CVA | | CI | | CVAI | | Diag-Ratio | |
|---|---|---|---|---|---|---|---|---|---|---|---|---|---|---|---|
| | | M | F | M | F | M | F | M | F | M | F | M | F | M | F |
| 0-3 mo | 3 | 35.9 | 35.4 | 9.2 | 9.0 | 11.7 | 11.5 | -0.2 | -0.2 | 70.4 | 70.1 | -1.3 | -1.4 | 0.9 | 0.9 |
| | 10 | 37.1 | 36.5 | 9.7 | 9.5 | 12.1 | 11.9 | -0.1 | -0.1 | 73.4 | 73.1 | -0.3 | -0.3 | 0.9 | 0.9 |
| | 25 | 38.2 | 37.6 | 10.1 | 9.9 | 12.5 | 12.3 | 0.1 | 0.1 | 76.2 | 75.8 | 0.7 | 0.6 | 1.0 | 1.0 |
| | 50 | 40.7 | 40.2 | 11.1 | 10.9 | 13.4 | 13.2 | 0.4 | 0.3 | 82.6 | 82.3 | 2.9 | 2.9 | 1.0 | 1.0 |
| | 75 | 43.3 | 42.7 | 12.1 | 11.9 | 14.3 | 14.1 | 0.6 | 0.6 | 88.1 | 88.8 | 5.2 | 5.1 | 1.0 | 1.0 |
| | 90 | 44.4 | 43.8 | 12.5 | 12.3 | 14.7 | 14.5 | 0.8 | 0.8 | 91.9 | 91.5 | 6.1 | 6.1 | 1.0 | 1.0 |
| | 97 | 45.6 | 45.0 | 12.9 | 12.7 | 15.1 | 15.0 | 0.9 | 0.9 | 94.8 | 94.5 | 7.2 | 7.1 | 1.0 | 1.0 |
| 4-6 mo | 3 | 37.4 | 36.8 | 9.6 | 9.4 | 12.2 | 12.0 | -0.2 | -0.2 | 69.7 | 69.3 | -1.6 | -1.7 | 0.9 | 0.9 |
| | 10 | 38.5 | 38.0 | 10.0 | 9.8 | 12.6 | 12.4 | -0.1 | -0.1 | 72.6 | 72.3 | -0.6 | -0.6 | 0.9 | 0.9 |
| | 25 | 39.6 | 39.1 | 10.5 | 10.2 | 13.0 | 12.8 | 0.1 | 0.0 | 75.4 | 75.0 | 0.6 | 0.5 | 1.0 | 1.0 |
| | 50 | 42.2 | 41.6 | 11.4 | 11.2 | 13.9 | 13.7 | 0.3 | 0.3 | 81.8 | 81.5 | 2.8 | 2.7 | 1.0 | 1.0 |
| | 75 | 44.7 | 44.1 | 12.4 | 12.2 | 14.9 | 14.7 | 0.6 | 0.6 | 88.7 | 88.4 | 5.0 | 4.9 | 1.0 | 1.0 |
| | 90 | 45.8 | 45.2 | 12.9 | 12.7 | 15.3 | 15.1 | 0.8 | 0.7 | 91.5 | 91.1 | 6.0 | 5.9 | 1.0 | 1.0 |
| | 97 | 47.0 | 46.4 | 13.3 | 13.1 | 15.7 | 15.6 | 0.9 | 0.9 | 94.4 | 94.1 | 7.0 | 7.0 | 1.0 | 1.0 |
| 7-9 mo | 3 | 38.8 | 38.2 | 9.9 | 9.7 | 12.7 | 12.5 | -0.2 | -0.2 | 69.3 | 68.9 | -1.7 | -1.8 | 0.9 | 0.9 |
| | 10 | 39.9 | 39.4 | 10.4 | 10.2 | 13.1 | 12.9 | -0.1 | -0.1 | 72.2 | 71.9 | -0.7 | -0.8 | 0.9 | 0.9 |
| | 25 | 41.0 | 40.5 | 10.8 | 10.6 | 13.5 | 13.3 | 0.1 | 0.0 | 75.0 | 74.6 | 0.4 | 0.3 | 1.0 | 1.0 |
| | 50 | 43.6 | 43.0 | 11.8 | 11.6 | 14.5 | 14.3 | 0.3 | 0.3 | 81.4 | 81.1 | 2.7 | 2.6 | 1.0 | 1.0 |
| | 75 | 46.1 | 45.5 | 12.8 | 12.6 | 15.4 | 15.2 | 0.6 | 0.6 | 87.9 | 87.5 | 4.9 | 4.8 | 1.0 | 1.0 |
| | 90 | 47.2 | 46.6 | 13.2 | 13.0 | 15.8 | 15.6 | 0.8 | 0.7 | 91.1 | 90.7 | 5.9 | 5.8 | 1.0 | 1.0 |
| | 97 | 48.4 | 47.8 | 13.7 | 13.5 | 16.2 | 16.0 | 0.9 | 0.9 | 94.0 | 93.7 | 6.9 | 6.8 | 1.0 | 1.0 |
| 10-12 mo | 3 | 40.2 | 39.6 | 10.3 | 10.1 | 13.2 | 13.1 | -0.2 | -0.2 | 68.8 | 68.5 | -1.9 | -1.9 | 0.9 | 0.9 |
| | 10 | 41.3 | 40.8 | 10.8 | 10.6 | 13.7 | 13.5 | -0.1 | -0.1 | 71.8 | 71.5 | -0.8 | -0.9 | 0.9 | 0.9 |
| | 25 | 42.4 | 41.9 | 11.2 | 11.0 | 14.1 | 13.9 | 0.1 | 0.0 | 74.6 | 74.2 | 0.3 | 0.2 | 1.0 | 1.0 |
| | 50 | 45.0 | 44.4 | 12.2 | 12.0 | 15.0 | 14.8 | 0.3 | 0.3 | 81.0 | 80.7 | 2.5 | 2.4 | 1.0 | 1.0 |
| | 75 | 47.5 | 47.0 | 13.2 | 13.0 | 15.9 | 15.7 | 0.6 | 0.6 | 87.5 | 87.1 | 4.6 | 4.5 | 1.0 | 1.0 |
| | 90 | 48.6 | 48.0 | 13.6 | 13.4 | 16.3 | 16.1 | 0.8 | 0.7 | 90.7 | 89.9 | 5.6 | 5.5 | 1.0 | 1.0 |
| | 97 | 49.8 | 49.2 | 14.1 | 13.8 | 16.7 | 16.5 | 0.9 | 0.9 | 93.6 | 92.9 | 6.7 | 6.7 | 1.0 | 1.0 |
| 13-15 mo | 3 | 41.6 | 41.0 | 10.7 | 10.5 | 13.8 | 13.6 | -0.2 | -0.2 | 68.4 | 68.1 | -2.0 | -2.1 | 0.9 | 0.9 |
| | 10 | 42.7 | 42.2 | 11.1 | 10.9 | 14.2 | 14.0 | -0.1 | -0.1 | 71.4 | 71.1 | -1.0 | -1.1 | 0.9 | 0.9 |
| | 25 | 43.8 | 43.3 | 11.6 | 11.4 | 14.6 | 14.4 | 0.0 | 0.0 | 74.2 | 74.2 | 0.0 | -0.1 | 1.0 | 1.0 |
| | 50 | 46.4 | 45.8 | 12.5 | 12.3 | 15.5 | 15.3 | 0.3 | 0.3 | 80.6 | 80.3 | 2.2 | 2.0 | 1.0 | 1.0 |
| | 75 | 48.9 | 48.4 | 13.5 | 13.3 | 16.5 | 16.3 | 0.6 | 0.6 | 87.1 | 86.7 | 4.4 | 4.3 | 1.0 | 1.0 |
| | 90 | 50.0 | 49.5 | 14.0 | 13.8 | 16.8 | 16.7 | 0.7 | 0.7 | 89.9 | 89.5 | 5.4 | 5.2 | 1.0 | 1.0 |
| | 97 | 51.2 | 50.6 | 14.4 | 14.2 | 17.3 | 17.1 | 0.9 | 0.9 | 92.8 | 92.5 | 6.5 | 6.4 | 1.0 | 1.0 |
| 16-18 mo | 3 | 43.0 | 42.4 | 11.0 | 10.8 | 14.3 | 14.1 | -0.2 | -0.2 | 68.0 | 67.7 | -2.2 | -2.2 | 0.9 | 0.9 |
| | 10 | 44.2 | 43.6 | 11.5 | 11.3 | 14.7 | 14.5 | -0.1 | -0.1 | 71.0 | 70.7 | -1.1 | -1.2 | 0.9 | 0.9 |
| | 25 | 45.3 | 44.7 | 11.9 | 11.7 | 15.3 | 15.5 | 0.0 | 0.0 | 73.8 | 73.4 | 0.0 | -0.2 | 1.0 | 1.0 |
| | 50 | 47.8 | 47.2 | 12.9 | 12.7 | 16.6 | 16.4 | 0.3 | 0.3 | 80.2 | 79.9 | 2.2 | 2.0 | 1.0 | 1.0 |
| | 75 | 50.3 | 49.8 | 13.9 | 13.7 | 17.5 | 16.8 | 0.6 | 0.6 | 86.7 | 86.3 | 4.4 | 4.3 | 1.0 | 1.0 |
| | 90 | 51.4 | 50.9 | 14.3 | 14.1 | 17.9 | 17.2 | 0.7 | 0.6 | 89.5 | 89.1 | 4.5 | 4.3 | 1.0 | 1.0 |
| | 97 | 52.6 | 52.0 | 14.8 | 14.6 | 18.3 | 17.6 | 0.9 | 0.8 | 92.4 | 92.1 | 5.4 | 5.2 | 1.0 | 1.0 |
| 19-21 mo | 3 | 44.4 | 43.8 | 11.4 | 11.2 | 14.6 | 15.2 | -0.2 | -0.2 | 67.6 | 67.3 | -2.3 | -2.4 | 0.9 | 0.9 |
| | 10 | 45.6 | 45.0 | 11.9 | 11.7 | 15.3 | 15.5 | -0.1 | -0.1 | 70.6 | 70.2 | -1.3 | -1.3 | 0.9 | 0.9 |
| | 25 | 46.7 | 46.1 | 12.3 | 12.1 | 15.7 | 16.0 | 0.0 | 0.0 | 73.4 | 73.0 | -0.3 | -0.4 | 1.0 | 1.0 |
| | 50 | 49.2 | 48.6 | 13.3 | 13.1 | 16.4 | 16.9 | 0.3 | 0.3 | 79.8 | 79.5 | 1.9 | 1.9 | 1.0 | 1.0 |
| | 75 | 51.7 | 51.2 | 14.3 | 14.1 | 17.5 | 17.9 | 0.6 | 0.6 | 86.3 | 85.9 | 4.2 | 4.1 | 1.0 | 1.0 |
| | 90 | 52.8 | 52.3 | 14.7 | 14.5 | 17.9 | 18.3 | 0.7 | 0.7 | 89.1 | 88.7 | 5.1 | 5.1 | 1.0 | 1.0 |
| | 97 | 54.0 | 53.4 | 15.2 | 15.0 | 18.1 | 18.7 | 0.9 | 0.8 | 92.4 | 91.7 | 6.2 | 6.1 | 1.0 | 1.0 |
| 22-24 mo | 3 | 45.8 | 45.2 | 11.8 | 11.6 | 15.4 | 15.3 | -0.1 | -0.2 | 67.6 | 67.3 | -2.3 | -2.4 | 0.9 | 0.9 |
| | 10 | 47.0 | 46.4 | 12.2 | 12.0 | 15.8 | 15.7 | 0.0 | -0.1 | 70.6 | 70.2 | -1.3 | -1.3 | 1.0 | 1.0 |
| | 25 | 48.1 | 47.5 | 12.7 | 12.5 | 16.2 | 16.0 | 0.1 | 0.0 | 73.4 | 73.0 | -0.3 | -0.4 | 1.0 | 1.0 |
| | 50 | 50.6 | 50.0 | 13.7 | 13.4 | 17.1 | 17.9 | 0.3 | 0.3 | 79.8 | 79.5 | 1.9 | 1.9 | 1.0 | 1.0 |
| | 75 | 53.2 | 52.6 | 14.6 | 14.4 | 18.0 | 17.9 | 0.5 | 0.6 | 86.3 | 85.9 | 4.2 | 4.1 | 1.0 | 1.0 |
| | 90 | 54.3 | 53.7 | 15.1 | 14.9 | 18.4 | 18.3 | 0.7 | 0.7 | 89.1 | 88.7 | 5.1 | 5.1 | 1.0 | 1.0 |
| | 97 | 55.4 | 54.9 | 15.5 | 15.3 | 18.7 | 18.7 | 0.8 | 0.8 | 92.4 | 91.7 | 6.2 | 6.1 | 1.0 | 1.0 |

# Fig.2'

**Percentiles during 2 years of Life in 748 healthy children**

| | | Circumference | | Width | |
|---|---|---|---|---|---|
| Group | Percentiles | M | F | M | F |
| 0-3 mo | 3 | 35,9 | 35,4 | 9,2 | 9,0 |
| | 10 | 37,1 | 36,5 | 9,7 | 9,5 |
| | 25 | 38,2 | 37,6 | 10,1 | 9,9 |
| | 50 | 40,7 | 40,2 | 11,1 | 10,9 |
| | 75 | 43,3 | 42,7 | 12,1 | 11,9 |
| | 90 | 44,4 | 43,8 | 12,5 | 12,3 |
| | 97 | 45,6 | 45,0 | 12,9 | 12,7 |
| 4-6 mo | 3 | 37,4 | 36,8 | 9,6 | 9,4 |
| | 10 | 38,5 | 38,0 | 10,0 | 9,8 |
| | 25 | 39,6 | 39,1 | 10,5 | 10,2 |
| | 50 | 42,2 | 41,6 | 11,4 | 11,2 |
| | 75 | 44,7 | 44,1 | 12,4 | 12,2 |
| | 90 | 45,8 | 45,2 | 12,9 | 12,7 |
| | 97 | 47,0 | 46,4 | 13,3 | 13,1 |
| 7-9 mo | 3 | 38,8 | 38,2 | 9,9 | 9,7 |
| | 10 | 39,9 | 39,4 | 10,4 | 10,2 |
| | 25 | 41,0 | 40,5 | 10,8 | 10,6 |
| | 50 | 43,6 | 43,0 | 11,8 | 11,6 |
| | 75 | 46,1 | 45,5 | 12,8 | 12,6 |
| | 90 | 47,2 | 46,6 | 13,2 | 13,0 |
| | 97 | 48,4 | 47,8 | 13,7 | 13,5 |
| 10-12 mo | 3 | 40,2 | 39,6 | 10,3 | 10,1 |
| | 10 | 41,3 | 40,8 | 10,8 | 10,6 |
| | 25 | 42,4 | 41,9 | 11,2 | 11,0 |
| | 50 | 45,0 | 44,4 | 12,2 | 12,0 |
| | 75 | 47,5 | 47,0 | 13,2 | 13,0 |
| | 90 | 48,6 | 48,0 | 13,6 | 13,4 |
| | 97 | 49,8 | 49,2 | 14,1 | 13,8 |

| Group | Percentiles | Circumference | | Width | |
|---|---|---|---|---|---|
| | | M | F | M | F |
| 13-15 mo | 3 | 41,6 | 41,0 | 10,7 | 10,5 |
| | 10 | 42,7 | 42,2 | 11,1 | 10,9 |
| | 25 | 43,8 | 43,3 | 11,6 | 11,4 |
| | 50 | 46,4 | 45,8 | 12,5 | 12,3 |
| | 75 | 48,9 | 48,4 | 13,5 | 13,3 |
| | 90 | 50,0 | 49,5 | 14,0 | 13,8 |
| | 97 | 51,2 | 50,6 | 14,4 | 14,2 |
| 16-18 mo | 3 | 43,0 | 42,4 | 11,0 | 10,8 |
| | 10 | 44,2 | 43,6 | 11,5 | 11,3 |
| | 25 | 45,3 | 44,7 | 11,9 | 11,7 |
| | 50 | 47,8 | 47,2 | 12,9 | 12,7 |
| | 75 | 50,3 | 49,8 | 13,9 | 13,7 |
| | 90 | 51,4 | 50,9 | 14,3 | 14,1 |
| | 97 | 52,6 | 52,0 | 14,8 | 14,6 |
| 19-21 mo | 3 | 44,4 | 43,8 | 11,4 | 11,2 |
| | 10 | 45,6 | 45,0 | 11,9 | 11,7 |
| | 25 | 46,7 | 46,1 | 12,3 | 12,1 |
| | 50 | 49,2 | 48,6 | 13,3 | 13,1 |
| | 75 | 51,7 | 51,2 | 14,3 | 14,1 |
| | 90 | 52,8 | 52,3 | 14,7 | 14,5 |
| | 97 | 54,0 | 53,4 | 15,2 | 15,0 |
| 22-24 mo | 3 | 45,8 | 45,2 | 11,8 | 11,6 |
| | 10 | 47,0 | 46,4 | 12,2 | 12,0 |
| | 25 | 48,1 | 47,5 | 12,7 | 12,5 |
| | 50 | 50,6 | 50,0 | 13,7 | 13,4 |
| | 75 | 53,2 | 52,6 | 14,6 | 14,4 |
| | 90 | 54,3 | 53,7 | 15,1 | 14,9 |
| | 97 | 55,4 | 54,9 | 15,5 | 15,3 |

| Group | Percentiles | Length | | CVA | |
|---|---|---|---|---|---|
| | | M | F | M | F |
| 0-3 mo | 3 | 11,7 | 11,5 | -0,2 | -0,2 |
| | 10 | 12,1 | 11,9 | -0,1 | -0,1 |
| | 25 | 12,5 | 12,3 | 0,1 | 0,1 |
| | 50 | 13,4 | 13,2 | 0,4 | 0,3 |
| | 75 | 14,3 | 14,1 | 0,6 | 0,6 |
| | 90 | 14,7 | 14,5 | 0,8 | 0,8 |
| | 97 | 15,1 | 15,0 | 0,9 | 0,9 |
| 4-6 mo | 3 | 12,2 | 12,0 | -0,2 | -0,2 |
| | 10 | 12,6 | 12,4 | -0,1 | -0,1 |
| | 25 | 13,0 | 12,8 | 0,1 | 0,0 |
| | 50 | 13,9 | 13,7 | 0,3 | 0,3 |
| | 75 | 14,9 | 14,7 | 0,6 | 0,6 |
| | 90 | 15,3 | 15,1 | 0,8 | 0,7 |
| | 97 | 15,7 | 15,5 | 0,9 | 0,9 |
| 7-9 mo | 3 | 12,7 | 12,5 | -0,2 | -0,2 |
| | 10 | 13,1 | 12,9 | -0,1 | -0,1 |
| | 25 | 13,5 | 13,3 | 0,1 | 0,0 |
| | 50 | 14,5 | 14,3 | 0,3 | 0,3 |
| | 75 | 15,4 | 15,2 | 0,6 | 0,6 |
| | 90 | 15,8 | 15,6 | 0,8 | 0,7 |
| | 97 | 16,2 | 16,0 | 0,9 | 0,9 |
| 10-12 mo | 3 | 13,2 | 13,1 | -0,2 | -0,2 |
| | 10 | 13,7 | 13,5 | -0,1 | -0,1 |
| | 25 | 14,1 | 13,9 | 0,0 | 0,0 |
| | 50 | 15,0 | 14,8 | 0,3 | 0,3 |
| | 75 | 15,9 | 15,7 | 0,6 | 0,6 |
| | 90 | 16,3 | 16,1 | 0,7 | 0,7 |
| | 97 | 16,7 | 16,5 | 0,9 | 0,9 |

| Group | Percentiles | CI | | CVAI | | Diag-Ratio | |
|---|---|---|---|---|---|---|---|
| | | M | F | M | F | M | F |
| 0-3 mo | 3 | 70,4 | 70,1 | -1,3 | -1,4 | 0,9 | 0,9 |
| | 10 | 73,4 | 73,1 | -0,3 | -0,3 | 0,9 | 0,9 |
| | 25 | 76,2 | 75,8 | 0,7 | 0,6 | 1,0 | 1,0 |
| | 50 | 82,6 | 82,3 | 2,9 | 2,9 | 1,0 | 1,0 |
| | 75 | 89,1 | 88,8 | 5,2 | 5,1 | 1,0 | 1,0 |
| | 90 | 91,9 | 91,5 | 6,1 | 6,1 | 1,0 | 1,0 |
| | 97 | 94,8 | 94,5 | 7,2 | 7,1 | 1,0 | 1,0 |
| 4-6 mo | 3 | 70,1 | 69,7 | -1,4 | -1,5 | 0,9 | 0,9 |
| | 10 | 73,0 | 72,7 | -0,4 | -0,5 | 0,9 | 0,9 |
| | 25 | 75,8 | 75,4 | 0,6 | 0,5 | 1,0 | 1,0 |
| | 50 | 82,2 | 81,9 | 2,8 | 2,7 | 1,0 | 1,0 |
| | 75 | 88,7 | 88,4 | 5,0 | 5,0 | 1,0 | 1,0 |
| | 90 | 91,5 | 91,1 | 6,0 | 5,9 | 1,0 | 1,0 |
| | 97 | 94,4 | 94,1 | 7,0 | 7,0 | 1,0 | 1,0 |
| 7-9 mo | 3 | 69,7 | 69,3 | -1,6 | -1,7 | 0,9 | 0,9 |
| | 10 | 72,6 | 72,3 | -0,6 | -0,6 | 0,9 | 0,9 |
| | 25 | 75,4 | 75,0 | 0,4 | 0,3 | 1,0 | 1,0 |
| | 50 | 81,8 | 81,5 | 2,7 | 2,6 | 1,0 | 1,0 |
| | 75 | 88,3 | 87,9 | 4,9 | 4,8 | 1,0 | 1,0 |
| | 90 | 91,1 | 90,7 | 5,9 | 5,8 | 1,0 | 1,0 |
| | 97 | 94,0 | 93,7 | 6,9 | 6,8 | 1,0 | 1,0 |
| 10-12 mo | 3 | 69,3 | 68,9 | -1,7 | -1,8 | 0,9 | 0,9 |
| | 10 | 72,2 | 71,9 | -0,7 | -0,8 | 0,9 | 0,9 |
| | 25 | 75,0 | 74,6 | 0,3 | 0,2 | 1,0 | 1,0 |
| | 50 | 81,4 | 81,1 | 2,5 | 2,4 | 1,0 | 1,0 |
| | 75 | 87,9 | 87,5 | 4,7 | 4,7 | 1,0 | 1,0 |
| | 90 | 90,7 | 90,3 | 5,7 | 5,6 | 1,0 | 1,0 |
| | 97 | 93,6 | 93,3 | 6,7 | 6,7 | 1,0 | 1,0 |

| Group | Percentiles | Circumference | | Width | |
|---|---|---|---|---|---|
| | | M | F | M | F |
| **13-15 mo** | 3 | 41,6 | 41,0 | 10,7 | 10,5 |
| | 10 | 42,7 | 42,2 | 11,1 | 10,9 |
| | 25 | 43,8 | 43,3 | 11,6 | 11,4 |
| | 50 | 46,4 | 45,8 | 12,5 | 12,3 |
| | 75 | 48,9 | 48,4 | 13,5 | 13,3 |
| | 90 | 50,0 | 49,5 | 14,0 | 13,8 |
| | 97 | 51,2 | 50,6 | 14,4 | 14,2 |
| **16-18 mo** | 3 | 43,0 | 42,4 | 11,0 | 10,8 |
| | 10 | 44,2 | 43,6 | 11,5 | 11,3 |
| | 25 | 45,3 | 44,7 | 11,9 | 11,7 |
| | 50 | 47,8 | 47,2 | 12,9 | 12,7 |
| | 75 | 50,3 | 49,8 | 13,9 | 13,7 |
| | 90 | 51,4 | 50,9 | 14,3 | 14,1 |
| | 97 | 52,6 | 52,0 | 14,8 | 14,6 |
| **19-21 mo** | 3 | 44,4 | 43,8 | 11,4 | 11,2 |
| | 10 | 45,6 | 45,0 | 11,9 | 11,7 |
| | 25 | 46,7 | 46,1 | 12,3 | 12,1 |
| | 50 | 49,2 | 48,6 | 13,3 | 13,1 |
| | 75 | 51,7 | 51,2 | 14,3 | 14,1 |
| | 90 | 52,8 | 52,3 | 14,7 | 14,5 |
| | 97 | 54,0 | 53,4 | 15,2 | 15,0 |
| **22-24 mo** | 3 | 45,8 | 45,2 | 11,8 | 11,6 |
| | 10 | 47,0 | 46,4 | 12,2 | 12,0 |
| | 25 | 48,1 | 47,5 | 12,7 | 12,5 |
| | 50 | 50,6 | 50,0 | 13,7 | 13,4 |
| | 75 | 53,2 | 52,6 | 14,6 | 14,4 |
| | 90 | 54,3 | 53,7 | 15,1 | 14,9 |
| | 97 | 55,4 | 54,9 | 15,5 | 15,3 |

| Group | Percentiles | Length | | CVA | |
|---|---|---|---|---|---|
| | | M | F | M | F |
| **13-15 mo** | 3 | 13,8 | 13,6 | -0,2 | -0,2 |
| | 10 | 14,2 | 14,0 | -0,1 | -0,1 |
| | 25 | 14,6 | 14,4 | 0,0 | 0,0 |
| | 50 | 15,5 | 15,3 | 0,3 | 0,3 |
| | 75 | 16,5 | 16,3 | 0,6 | 0,6 |
| | 90 | 16,8 | 16,7 | 0,7 | 0,7 |
| | 97 | 17,3 | 17,1 | 0,9 | 0,9 |
| **16-18 mo** | 3 | 14,3 | 14,1 | -0,2 | -0,2 |
| | 10 | 14,7 | 14,5 | -0,1 | -0,1 |
| | 25 | 15,1 | 14,9 | 0,0 | 0,0 |
| | 50 | 16,1 | 15,9 | 0,3 | 0,3 |
| | 75 | 17,0 | 16,8 | 0,6 | 0,6 |
| | 90 | 17,4 | 17,2 | 0,7 | 0,7 |
| | 97 | 17,8 | 17,6 | 0,9 | 0,8 |
| **19-21 mo** | 3 | 14,8 | 14,6 | -0,2 | -0,2 |
| | 10 | 15,3 | 15,1 | -0,1 | -0,1 |
| | 25 | 15,7 | 15,5 | 0,0 | 0,0 |
| | 50 | 16,6 | 16,4 | 0,3 | 0,3 |
| | 75 | 17,5 | 17,3 | 0,6 | 0,6 |
| | 90 | 17,9 | 17,7 | 0,7 | 0,7 |
| | 97 | 18,3 | 18,1 | 0,9 | 0,8 |
| **22-24 mo** | 3 | 15,4 | 15,2 | -0,2 | -0,2 |
| | 10 | 15,8 | 15,6 | -0,1 | -0,1 |
| | 25 | 16,2 | 16,0 | 0,0 | 0,0 |
| | 50 | 17,1 | 16,9 | 0,3 | 0,3 |
| | 75 | 18,0 | 17,9 | 0,6 | 0,6 |
| | 90 | 18,4 | 18,3 | 0,7 | 0,7 |
| | 97 | 18,9 | 18,7 | 0,8 | 0,8 |

h

| Group | Percentiles | CI | | CVAI | | Diag-Ratio | |
|---|---|---|---|---|---|---|---|
| | | M | F | M | F | M | F |
| 13-15 mo | 3 | 68,8 | 68,5 | -1,9 | -1,9 | 0,9 | 0,9 |
| | 10 | 71,8 | 71,5 | -0,8 | -0,9 | 0,9 | 0,9 |
| | 25 | 74,6 | 74,2 | 0,1 | 0,1 | 1,0 | 1,0 |
| | 50 | 81,0 | 80,7 | 2,4 | 2,3 | 1,0 | 1,0 |
| | 75 | 87,5 | 87,1 | 4,6 | 4,5 | 1,0 | 1,0 |
| | 90 | 90,3 | 89,9 | 5,6 | 5,5 | 1,0 | 1,0 |
| | 97 | 93,2 | 92,9 | 6,6 | 6,5 | 1,0 | 1,0 |
| 16-18 mo | 3 | 68,4 | 68,1 | -2,0 | -2,1 | 0,9 | 0,9 |
| | 10 | 71,4 | 71,1 | -1,0 | -1,1 | 0,9 | 0,9 |
| | 25 | 74,2 | 73,8 | 0,0 | -0,1 | 1,0 | 1,0 |
| | 50 | 80,6 | 80,3 | 2,2 | 2,2 | 1,0 | 1,0 |
| | 75 | 87,1 | 86,7 | 4,5 | 4,4 | 1,0 | 1,0 |
| | 90 | 89,9 | 89,5 | 5,4 | 5,4 | 1,0 | 1,0 |
| | 97 | 92,8 | 92,5 | 6,5 | 6,4 | 1,0 | 1,0 |
| 19-21 mo | 3 | 68,0 | 67,7 | -2,2 | -2,2 | 0,9 | 0,9 |
| | 10 | 71,0 | 70,7 | -1,1 | -1,2 | 1,0 | 1,0 |
| | 25 | 73,8 | 73,4 | -0,2 | -0,2 | 1,0 | 1,0 |
| | 50 | 80,2 | 79,9 | 2,1 | 2,0 | 1,0 | 1,0 |
| | 75 | 86,7 | 86,3 | 4,3 | 4,3 | 1,0 | 1,0 |
| | 90 | 89,5 | 89,1 | 5,3 | 5,2 | 1,0 | 1,0 |
| | 97 | 92,4 | 92,1 | 6,3 | 6,2 | 1,0 | 1,0 |
| 22-24 mo | 3 | 67,6 | 67,3 | -2,3 | -2,4 | 0,9 | 0,9 |
| | 10 | 70,6 | 70,2 | -1,3 | -1,3 | 1,0 | 1,0 |
| | 25 | 73,4 | 73,0 | -0,3 | -0,4 | 1,0 | 1,0 |
| | 50 | 79,8 | 79,5 | 1,9 | 1,9 | 1,0 | 1,0 |
| | 75 | 86,3 | 85,9 | 4,2 | 4,1 | 1,0 | 1,0 |
| | 90 | 89,1 | 88,7 | 5,1 | 5,1 | 1,0 | 1,0 |
| | 97 | 92,0 | 91,7 | 6,2 | 6,1 | 1,0 | 1,0 |

**Europäisches Patentamt**
**European Patent Office**
**Office européen des brevets**

# EUROPÄISCHER RECHERCHENBERICHT

Nummer der Anmeldung

EP 16 17 6087

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | Dulcey Lima: "Asymmetrical Brachycephaly: A New Classification of Head Shape Deformity in Young Babies Dulcey Lima CO, OTR/L Orthomerica Products", , 30. Dezember 2010 (2010-12-30), XP055322335, Gefunden im Internet: URL:https://web.archive.org/web/20101230013537/http://www.oandp.org/publications/jop/2009/2009-07.pdf [gefunden am 2016-11-23] * "Review of the literature", "Measurement Instrument" & legende zu den Bilder; Seite 1 - Seite 3 * ----- | 1-4 | INV. G06T7/00 A61B5/107 A61B5/00 |
| X | Anonymous: "actionorthosante.ca/english", , 24. Januar 2015 (2015-01-24), XP055322347, Gefunden im Internet: URL:https://web.archive.org/web/20150124040825/http://actionorthosante.ca/english/Plagiocephaly-Severity-Scale.php [gefunden am 2016-11-23] * Seite 2 * ----- | 1-4 | |

RECHERCHIERTE SACHGEBIETE (IPC)

G06T
A61B

Der vorliegende Recherchenbericht wurde für alle Patentansprüche erstellt

| Recherchenort | Abschlußdatum der Recherche | Prüfer |
|---|---|---|
| Den Haag | 30. November 2016 | Almeida, Mariana |